# EUROPÄISCHE PATENTSCHRIFT

(11) **EP 3 392 237 B1**
(45) Veröffentlichungstag und Bekanntmachung des Hinweises auf die Patenterteilung: **02.10.2019**
(21) Anmeldenummer: 17167628.1
(22) Anmeldetag: 21.04.2017
(51) Int. Cl.: C07C 253/10, C07C 253/34, C07F 9/30, C07F 9/32, C07C 255/15

(54) **VERFAHREN ZUR HERSTELLUNG VON ACROLEINCYANHYDRINEN**
METHOD FOR MANUFACTURING ACROLEIN CYANOHYDRINS
PROCÉDÉ DESTINÉ À LA FABRICATION DE CYANHYDRINE DE L'ACROLÉINE

(43) Veröffentlichungstag der Anmeldung: 24.10.2018
(73) Patentinhaber: Evonik Degussa GmbH, 45128 Essen (DE)
(72) Erfinder: OMEIS, Marianne, 46286 Dorsten (DE)
(74) Vertreter: Evonik Patent Association

(56) Entgegenhaltungen:
- WO-A1-2015/173146
- DE-A1- 3 047 024
- US-A- 3 850 976
- US-A- 4 218 393
- US-A- 4 859 784

## Beschreibung

Die vorliegende Erfindung betrifft ein verbessertes Verfahren zur Herstellung von Acroleincyanhydrinen aus Blausäure und den entsprechenden Acroleinen. Das Verfahren zeichnet sich dadurch aus, dass die erhaltenen Acroleincyanhydrine einen sehr geringen Anteil an Blausäure aufweisen bzw. blausäurefrei sind und sich deshalb besonders gut als Zwischenprodukte für die Synthese von Glufosinaten eignen.

### Hintergrund der Erfindung

Acroleincyanhydrine sind industriell wichtige Rohstoffe.

Eine wichtige Anwendung dieser Verbindungen ist die Synthese von Phosphinotricin (2-Amino-4-[hydroxy(methyl))phosphinoyl]-Buttersäure, vulgo "Glufosinat", bzw. Gulfonsinatsalzen (EP 0 546 566 A1).

Verfahren zur Herstellung der Glufosinate und ähnlicher Verbindungen beschreiben US 4,521,348, US 4,599,207, US 6,359,162 B1, CN 102372739 A, CN 102399240 A, CN 101830926 A.

DE 23 02 523 A1 nennt ein ähnliches Verfahren, in dem Ethan-1,2-diphosphinsäurediester aus Phosphonigsäureester und Acetylen erhalten wird. Ähnliche, Phosphonylgruppen aufweisende Verbindungen und deren Herstellung beschreibt S. R. Piettre, Tetrahedron Letters, 37 (13), 2233 - 2236.

Glufosinate werden hauptsächlich als Herbizide eingesetzt (EP 0 377 870 A1, US 4,168,963). Daneben nennt die EP 0 029 168 A1 die Anwendung ähnlicher Phosphorverbindungen zur Herstellung von Copolymeren auf dem Gebiet der Photographie.

Ein wesentlicher Schritt in der Synthese der Glufosinate ist die Addition eines Phosphinsäureesters an die Doppelbindung des Acroleincyanhydrins (im Folgenden "AcCH") bzw. des Acroleincyanhydrinacetats ("ACA"). In dieser Reaktion wird in vielen Verfahren des Standes der Technik das durch eine Acetatgruppe geschützte ACA, erhältlich nach Verfahren gemäß EP 0 019 227 A1, eingesetzt, z.B. in EP 0 011 245 A1, EP 0 127 877 A2. Die Reaktion mit dem geschützten Derivat vermeidet unerwünschte Nebenreaktionen. Andererseits führt sie zu weiteren Nachteilen, wie im Stand der Technik beschrieben: WO 2015/173146 A1 diskutiert die Nebenprodukte, die mit der Acetylierung der OH-Funktion des Cyanhydrins bzw. dem Verzicht darauf zusammenhängen (WO 2015/173146 A1, Seite 4, Zeilen 8 - 24).

Daneben wurde aber schon in der DE 30 47 024 A1 erkannt, dass auch das an der OH-Gruppe ungeschützte AcCH in dieser Reaktion eingesetzt werden kann. Gerade wenn dieses ungeschützte AcCH anstatt ACA als Edukt eingesetzt wird, ist eine möglichst hohe Reinheit desselben sehr wichtig.

AcCH wird üblicherweise durch Reaktion von Acrolein und Blausäure gewonnen (US 3,850,976). Dabei tritt das Problem auf, dass sich stets ein gewisser Rest des Edukts Blausäure im erhaltenen AcCH wiederfindet. Dies ist erst recht dann der Fall, wenn ein Überschuss an Blausäure bezogen auf das Acrolein eingesetzt wird, was aufgrund der gewünschten möglichst vollständigen Umsetzung des Acroleins üblich ist.

Dieser Restgehalt an Blausäure verursacht aber mehrere Probleme: So führt er zu unerwünschten Nebenreaktionen in der folgenden Synthesesequenz. Dies ist gerade bei der Synthese der Glufosinate problematisch, worauf schon im Stand der Technik (WO 2015/173146 A1: Seite 2, Zeile 15) hingewiesen wird. Außerdem ist Blausäure toxisch und kann explosionsartig reagieren, besonders da im nachfolgenden Schritt ein Peroxid zugegen ist, was zur Sauerstofffreisetzung führt. Es ist daher auch unter dem Gesichtspunkt der Arbeitssicherheit unerwünscht, dass freie Blausäure im AcCH vorhanden ist.

Der Stand der Technik beschreibt mehrere Verfahren zum Entfernen der Blausäure - allerdings sind diese recht umständlich oder verursachen weitere Probleme. Der Grund dafür ist, dass die Behandlung von AcCH problematisch ist und dieses aufgrund seiner Instabilität nicht einfach den üblichen Reinigungsmethoden unterzogen werden kann.

Diese bisherige Bereitstellung von blausäurefreiem AcCH erfordert nach herkömmlichen Verfahren folgende Schritte:
a) Acetylierung des mit Blausäure verunreinigten Roh-AcCHs zu ACA und Aufreinigung (beschrieben zum Beispiel in EP 0 019 750 A1);
b) Deacetylierung des ACAs via lonenaustausch (wie in Beispiel 1 der WO 2015/173146 A1, Seite 17, Zeilen 12 - 22 beschrieben).

Dies bedeutet, dass bisher zur Bereitstellung von blausäurefreiem AcCH die Acetylierung nicht umgangen werden kann. Die sowohl bei der Acetylierung als auch bei der Deacetylierung frei werdende Essigsäure führt aber ihrerseits zu weiteren Verunreinigungen im nach der Deacetylierung erhaltenen AcCH. Diese Verunreinigungen können wieder zu Nebenreaktionen in den Folgeschritten der Synthese führen.

Ein weiterer Nachteil der vorbeschriebenen Blausäureentfernung ist, dass dies durch die teure Verwendung eines Ionenaustauschers sehr aufwendig ist.

Es besteht das Bedürfnis, besonders in der Synthese von Glufosinaten, auf die Acetylierung des (Zwischen-)Produktes AcCH zu verzichten, und dieses dennoch möglichst frei von Blausäure einsetzen zu können.

Aufgabe der vorliegenden Erfindung war demnach, einen gegenüber dem Stand der Technik verbesserten Syntheseweg zur Herstellung von Acroleincyanhydrin und ähnlichen Verbindungen bereit zu stellen. Diese Synthese sollte vor allem zu Produkten mit geringeren Verunreinigungen, insbesondere einem möglichst geringen Anteil an Blausäure, führen, und gleichzeitig die Nachteile der Verfahren des Standes der Technik vermeiden.

Die Erfindung löst diese Aufgabe und zeigt überraschend einen effizienten alternativen Weg auf, diese Nebenprodukte zu entfernen und leichter zu reinem AcCH zu gelangen.

### Detaillierte Beschreibung der Erfindung

Die vorliegende Erfindung betrifft demnach ein Verfahren zur Herstellung einer Verbindung der Formel **(I)** 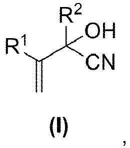 wobei R¹ und R² jeweils Wasserstoff sind,
umfassend die folgenden Schritte:
(a) Umsetzen mindestens einer Verbindung der Formel **(II)** 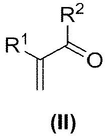 mit Blausäure und mindestens einer Base **B** ausgewählt aus der Gruppe bestehend aus Ammoniak, Trialkylamin, Ammoniumsalz, wodurch ein Rohprodukt **RP**, welches **(I)** und Blausäure umfasst, erhalten wird;
b) mindestens teilweise Entfernung von Blausäure aus dem Rohprodukt **RP,** in dem dieses einer Strippung unterworfen wird, wodurch ein Reinprodukt umfassend die Verbindung **(I)**, wobei das Reinprodukt einen gegenüber **RP** verringerten Gehalt an Blausäure aufweist, erhalten wird,
dadurch gekennzeichnet, dass die Strippung bei einem Druck im Bereich von 10 bis 500 mbar durchgeführt wird, wobei bei der Strippung in Schritt b) eine Temperatur von ≤ 85 °C eingehalten wird, wobei gegebenenfalls ein inertes Strippungsgas im Gegenstrom eingesetzt wird.

R¹ und R² sind jeweils Wasserstoff. Es sind R¹ = R² = Wasserstoff - dann handelt es sich bei der Verbindung der Formel **(II)** um Acrolein und bei der Verbindung der Formel **(I)** um Acroleincyanhydrin.

Im Schritt a) des erfindungsgemäßen Verfahrens wird eine Verbindung der Formel **(II)** 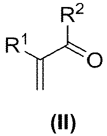 mit Blausäure und einer Base **B** umgesetzt, wodurch ein Rohprodukt **RP**, welches **(I)** und Blausäure umfasst, erhalten wird.

Die Reaktionsbedingungen dazu sind dem Fachmann bekannt und können beispielsweise der US 3,850,976 entnommen werden.

Die Blausäure kann als Flüssigkeit oder gasförmig, bevorzugt als Flüssigkeit eingesetzt werden.

Die Umsetzung der Verbindung der Struktur **(II)** kann in einem Reaktor mit Wälzung stattfinden. Die Wälzung stellt eine intensive Vermischung der kritischen Reaktanden sicher.

Die Base **B** ist aus der Gruppe bestehend aus Ammoniak, Trialkylamin, Ammoniumsalz auszuwählen, bevorzugter aus der Gruppe bestehend aus Ammoniak, Trialkylamin auszuwählen, noch bevorzugter aus der Gruppe bestehend aus Ammoniak, Triethylamin, am bevorzugtesten Triethylamin, auszuwählen. Der Ammoniak kann in einer Mischung mit CO₂ eingesetzt werden, wie in der US 3,850,976 beschrieben.

Bevorzugtes Trialkylamin ist Triethylamin.

Bevorzugte Ammoniumsalze sind Ammoniumcarbonat, Ammoniumhydrogencarbonat, Ammoniumcarbamat, besonders bevorzugt Ammoniumcarbonat, Ammoniumhydrogencarbonat.

Der Schritt a) wird dabei insbesondere bei einer Temperatur von - 50 °C bis 80 °C durchgeführt, bevorzugt bei einer Temperatur von - 40 °C bis 60 °C durchgeführt, bevorzugter bei einer Temperatur von -30 °C bis 30 °C durchgeführt, noch bevorzugter bei einer Temperatur von - 10 bis 10 °C durchgeführt, noch mehr bevorzugter bei einer Temperatur von - 5 °C bis 5 °C durchgeführt.

Die Menge an eingesetzter Blausäure in Schritt a) des erfindungsgemäßen Verfahrens ist grundsätzlich nicht beschränkt. Um jedoch eine möglichst vollständige Umsetzung der Verbindung **(II)** zu gewährleisten, und da die vorliegende Erfindung ja gerade die leichte Entfernung überschüssiger Blausäure im **RP** erlaubt, wird die Blausäure in Schritt a) des erfindungsgemäßen Verfahrens insbesondere in einer Menge von > 1 molaren Äquivalent, bevorzugt in einer Menge von > 1 bis 10 molaren Äquivalenten, bevorzugter in einer Menge von 1.01 molaren Äquivalenten bis 7.5 molaren Äquivalenten, noch bevorzugter 1.03 molaren Äquivalenten bis 5 molaren Äquivalenten, noch bevorzugter 1.3 molaren Äquivalenten bis 2 molaren Äquivalenten, jeweils bezogen auf die Menge aller in Schritt a) eingesetzten Verbindungen der Formel **(II)**, eingesetzt.

Die Menge an Base **B** in Schritt a) ist nicht weiter beschränkt. Bevorzugt beträgt die Menge an Base **B** in Schritt a) 0.01 bis 5 Gew.-%, bevorzugter 0.1 bis 1 Gew-% bezogen auf die Summe der Gewichte der in Schritt a) eingesetzten Blausäure und aller in Schritt a) eingesetzten Verbindungen der Formel **(II)**.

Noch bevorzugter wird in Schritt a) so viel Base **B** eingesetzt, dass die Reaktionsmischung im Schritt a) einen pH-Wert von ≥ 6.9, bevorzugter 7.0 bis 7.5, bevorzugt 7.3 aufweist (bei 25 °C).

Der pH-Wert der Reaktionsmischung in Schritt a) kann mit entsprechenden Elektroden routinemäßig vom Fachmann bestimmt werden, so dass er darüber die Menge der nötigen Base **B** bestimmen kann. Eine mögliche pH-Elektrode ist zum Beispiel die "Flushtrode pH Elektrode A238060" (Hersteller: Hamilton).

Die Reaktionszeit ist nicht weiter beschränkt. Man führt die Umsetzung in Schritt a) durch, bis der gewünschte Umsatz der Verbindung der Formel **(II)** mit Blausäure zum Produkt **(I)** erreicht ist. Insbesondere liegt die Reaktionszeit bei 20 Sekunden bis 3 Stunden, bevorzugt 1 bis 85 Minuten.

Schritt a) des erfindungsgemäßen Verfahrens wird mit oder ohne, bevorzugt ohne Lösungsmittel durchgeführt. Wird ein Lösungsmittel eingesetzt, ist dies bevorzugt mindestens eines ausgewählt aus Alkohol, Toluol, Xylol, Methylenchlorid, Dialkylformamid oder Dialkylsulfoxid, noch bevorzugter mindestens eines ausgewählt aus Methanol, Ethanol, Toluol, Xylol, Methylenchlorid.

Am Ende des Schrittes a) erhält man ein **RP**, welches die Zielverbindung **(I)** umfasst, und daneben auch Blausäure umfasst. Der Anteil an Blausäure ist abhängig vom Überschuss an Blausäure, der in Schritt a) des erfindungsgemäßen Verfahrens eingesetzt wurde und auch von der Umsetzung in Schritt a). Insbesondere beträgt der Anteil an Blausäure im **RP** bezogen auf die Menge der von **RP** umfassten Verbindung der Formel **(I)** bis zu 10 Gew.-%, liegt bevorzugt im Bereich von 3 bis 4 Gew.-%.

Dieses Rohprodukt **RP** kann nun dem Schritt b), das heißt der erfindungsgemäßen Strippung, zugeführt werden.

Es ist jedoch von Vorteil, dass das **RP** vor Durchführung des Schrittes b) stabilisiert wird. Stabilisierung bedeutet erfindungsgemäß, dass das Rohprodukt **RP** mit mindestens einer Säure vermischt wird, so dass es nach Vermischen mit der Säure einen pH-Wert von < 6.9 aufweist, bevorzugt einen pH von < 5.0, bevorzugter < 4.0, sogar bevorzugter < 3.0, noch bevorzugter < 2.0, noch mehr bevorzugter < 1.0 (bei 25 °C) aufweist.

Der pH-Wert der so stabilisierten Mischung kann mit entsprechenden Elektroden routinemäßig vom Fachmann bestimmt werden, so dass er darüber die Menge der nötigen Säure bestimmen kann. Eine mögliche pH-Elektrode ist zum Beispiel die "Flushtrode pH Elektrode A238060" (Hersteller: Hamilton).

Die Menge der zur Stabilisierung mit dem **RP** vermischten Säuren liegt vorzugsweise bei ≤ 10 Gew.-%, bevorzugt bei ≤ 5 Gew.-%, bevorzugter bei ≤ 1 Gew.-%, noch bevorzugter bei ≤ 0.1 Gew.-% bezogen auf die Menge aller Verbindungen der Formel **(I)** im **RP**.

Die dazu verwendete nötige Säure wird bevorzugt ausgewählt aus der Gruppe bestehend aus Mineralsäuren, Alkylcarbonsäure, aromatische Sulfonsäure, oder Mischungen aus aromatischer Sulfonsäure und Alkylcarbonsäure.

Eine bevorzugte Alkylcarbonsäure ist Essigsäure.

Eine bevorzugte Mineralsäure ist Phosphorsäure.

"Aromatische Sulfonsäure" bedeutet, dass der aromatische Rest in dieser Sulfonsäure Alkylsubstituenten tragen kann oder auch nicht. Bevorzugte aromatische Sulfonsäuren sind ausgewählt aus Benzolsulfonsäure und alkylsubstituierten Benzolsulfonsäuren, wobei die Alkylsubstituenten bevorzugt 1 bis 20 Kohlenstoffatome haben und der Phenylring bevorzugt nur einen *para*-Alkylsubstituenten trägt, wie z.B. *para*-Toluolsulfonsäure.

Werden Mischungen aus aromatischer Sulfonsäure und Alkylcarbonsäure zur Stabilisierung des **RP** eingesetzt, liegen insbesondere in diesen Mischungen alle aromatischen Sulfonsäuren und alle Alkylcarbonsäuren im folgendem Gewichtsverhältnis vor:
Gewicht aller aromatischen Sulfonsäuren zu allen Alkylcarbonsäuren = 9:1 bis 1:9, bevorzugt 8:2 bis 2:8, bevorzugter 7:3 bis 3:7, noch bevorzugter 6:4 bis 4:6, am bevorzugtesten 1:1.

Das nach Schritt a) erhaltene **RP,** sei es nun stabilisiert oder nicht weiter stabilisiert, bevorzugt ist es stabilisiert, wird dann dem Schritt b) unterworfen.

In Schritt b) findet die mindestens teilweise Entfernung von Blausäure aus dem Rohprodukt **RP** statt, in dem dieses einer Strippung unterworfen wird, wodurch ein Reinprodukt umfassend die Verbindung **(I)**, wobei das Reinprodukt einen gegenüber **RP** verringerten Gehalt an Blausäure aufweist, erhalten wird.

Dies war überraschend, denn Acroleincyanhydrin und ähnliche Verbindungen sind gerade in Kombination mit Blausäure sehr explosiv in Anwesenheit von Sauerstoff, wie es im Folgeschritt zu erwarten ist (Gestis-Stoffdatenbank des Instituts für Arbeitsschutz). Gemäß Fachwissen sind diese Mischungen einer destillativen Reinigung grundsätzlich nicht zugänglich, da eine saure Stabilisierung jederzeit sichergestellt sein muß und weiterhin die thermische Stabilität des Produktes nicht ausreicht, um eine Verdampfung unter technisch realisierbaren Vakuumbedingungen durchzuführen.

Im Verfahren gemäß der vorliegenden Erfindung wird das **RP** aus Schritt a) (stabilisiert oder unstabilisiert, bevorzugt stabilisiert) dann im Schritt b) einer Strippung unterzogen. Es wurde überraschend festgestellt, dass mit diesem Verfahren eine besonders schonende Entfernung der Blausäure möglich ist.

"Strippung" ist ein dem Fachmann bekanntes physikalisches Trennverfahren, was in vielen Gebieten des Standes der Technik zu Reinigung von Flüssigkeiten verwendet wird (zum Beispiel beschrieben in M. Kriebel: "Absorption, 2. Design of Systems and Equipment", Ullmann's Encyclopedia of Industrial Chemistry, Electronic Release, chap. 3, Wiley VCH, Weinheim October 2008). In diesem wird eine Gasphase im Gegenstrom mit einer zu reinigenden Phase oder die zu reinigende Phase bei vermindertem Druck von unter 1 bar mit oder ohne einem Gegenstrom kontaktiert. Diese Kontaktierung geschieht erfindungsgemäß insbesondere in einer Kolonne.

In der vorliegenden Erfindung wird die Strippung bei einem verminderten Druck von im Bereich von 10 bis 500 mbar durchgeführt. Es kann gegebenenfalls ein inertes Strippungsgas im Gegenstrom eingesetzt werden, was bedeutet, dass zur Unterstützung der Strippung ein solches inertes Strippungsgas in die Kolonne im Gegenstrom zum **RP** eingeführt werden kann, solange nur der Druck im Bereich von 10 bis 500 mbar bleibt.

"Wobei gegebenenfalls ein inertes Strippungsgas im Gegenstrom eingesetzt wird" bedeutet demnach, dass zur Unterstützung der Strippung ein solches inertes Strippungsgas in die Kolonne im Gegenstrom zu dem in die Kolonne eingeführten **RP** umfassend Blausäure und die Verbindung der Formel **(I)** eingeführt wird und dabei der Druck im Bereich von 10 bis 500 mbar bleibt, oder eben kein solches inertes Strippungsgas eingeführt wird.

Bevorzugt ist die Durchführung ohne inertes Strippungsgas.

Die Temperatur der Strippung kann durch Einstellung eines entsprechenden Unterdruckes in der Kolonne routinemäßig vom Fachmann eingestellt werden.

Der Druck liegt erfindungsgemäß im Schritt b) im Bereich 10 - 500 mbar, noch bevorzugter im Bereich 10 - 200 mbar.

Bevorzugt ist dabei, dass die Temperatur von 85 °C oder weniger bei der Strippung eingehalten wird, denn es wurde überraschend festgestellt, dass eine besonders schonende und vorteilhafte Reinigung von AcCH bei einer Temperatur von 85 °C oder tiefer möglich ist. Bevorzugt liegt die Temperatur bei ≤ 72.5 °C, bevorzugter in einem Bereich von - 10 °C bis 70 °C, noch bevorzugter in einem Bereich von - 5 °C bis 30 °C

Die Reinigung des **RP** kann durch ein Vergrößern der Oberfläche desselben verbessert werden. Vorzugsweise wird dazu das **RP** im Schritt b) des erfindungsgemäßen Verfahrens mindestens teilweise über eine Schüttung aus Füllkörpern oder über eine strukturierte Packung geleitet. Geeignet sind dazu alle Füllkörper und strukturierten Packungen, die dem Fachmann aus dem Stand der Technik für Destillationen und für Absorptionsprozesse bekannt sind. Alternativ kann die Strippung in einem Fallfilm- oder Dünnschichtverdampfer erfolgen. Diese Apparate sind dem Fachmann gemäß dem Stand der Technik bekannt.

Bevorzugtes inertes Strippungsgas ist Stickstoff.

Wird ein inertes Strippungsgas eingesetzt, sollte der Wassergehalt desselben gering sein, bevorzugt < 1 Vol.-%, bevorzugter < 0,1 Vol.-%.

In Schritt b) wird dann erfindungsgemäß ein Reinprodukt erhalten, welches gegenüber dem **RP** einen verringerten Gehalt an Blausäure aufweist. Typischerweise enthält dieses Reinprodukt einen Gehalt von < 0.1 Gew.-% Blausäure bezogen auf die vom Reinprodukt umfasste Verbindung der Formel **(I)**.

Bevorzugt schließt sich an den Schritt b) des erfindungsgemäßen Verfahrens dann noch ein weiterer Schritt c) an. Die vorliegende Erfindung betrifft demnach auch zusammen mit den vorher beschriebenen Schritten a) und b) ein Verfahren zur Herstellung einer Verbindung der Formel **(III)** 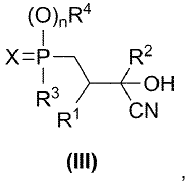
wobei R¹ und R² die vorgenannten Bedeutungen haben,
R³, R⁴ unabhängig voneinander jeweils ausgewählt sind aus (Halo)alkyl, (Halo)aryl, (Halo)aralkyl, (Halo)cycloalkyl,
X Sauerstoff oder Schwefel ist,
n= 0 oder 1;

umfassend die Schritte a) und b) gemäß dem erfindungsgemäßen Verfahren zur Herstellung einer Verbindung der Formel **(I)** und zusätzlich den Schritt c), in welchem das in Schritt b) erhaltene Reinprodukt umfassend die Verbindung **(I)**, wobei das Reinprodukt einen gegenüber **RP** verringerten Gehalt an Blausäure aufweist, mit einer Verbindung der Formel **(IV)** 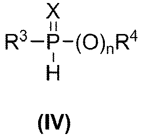 umgesetzt wird.

Diese Umsetzung kann nach dem Fachmann bekannten Verfahren (beschrieben in der WO 2015/173146 A1) erfolgen. Durch die Verwendung des in Schritt b) erhaltene Reinprodukts umfassend die Verbindung **(I)**, und einen verringerten Blausäuregehalt kommt es zu weniger Nebenreaktionen im Schritt c). Die Gefahr von Explosionen, welche durch Blausäure und radikalbildender Substanz in Schritt c) besteht, ist ebenfalls verringert.

Die Temperatur in der Umsetzung im Schritt c) liegt bevorzugt bei 50 °C bis 105 °C, bevorzugter bei 60 °C bis 95 °C, noch bevorzugter bei 65 °C bis 90 °C.

R³, R⁴ sind bevorzugt unabhängig voneinander jeweils ausgewählt aus (Halo)alkyl mit 1 bis 12 Kohlenstoffatomen, (Halo)aryl mit 6 bis 10 Kohlenstoffatomen, (Halo)aralkyl mit 7 bis 10 Kohlenstoffatomen, (Halo)cycloalkyl mit 4 bis 10 Kohlenstoffatomen.

(Halo)alkyl bedeutet erfindungsgemäß Alkyl oder Haloalkyl.
(Halo)aryl bedeutet erfindungsgemäß Aryl oder Haloaryl.
(Halo)aralkyl bedeutet erfindungsgemäß Aralkyl oder Haloaralkyl.
(Halo)cycloalkyl bedeutet erfindungsgemäß Cycloalkyl oder Halocycloalkyl.

R³ ist bevorzugt ausgewählt aus Alkyl mit 1 bis 4 Kohlenstoffatomen, Haloalkyl mit 1 bis 4 Kohlenstoffatomen, noch bevorzugter Methyl oder Ethyl, noch bevorzugter Methyl.

R⁴ ist bevorzugt ausgewählt aus Alkyl mit 1 bis 6 Kohlenstoffatomen, Haloalkyl mit 1 bis 6 Kohlenstoffatomen, bevorzugter ausgewählt aus Alkyl mit 3 bis 6 Kohlenstoffatomen, noch bevorzugter ausgewählt aus Alkyl mit 4 oder 5 Kohlenstoffatomen, am bevorzugtesten *n*-Butyl oder *n*-Pentyl.

Vorzugsweise wird in dem Schritt c) außerdem mindestens eine radikalbildende Substanz eingesetzt. Bei dieser handelt es sich bevorzugt um einen Radikalbildner der Formel **(V)** mit 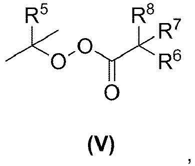
wobei R⁵ Methyl, Ethyl, 2,2-Dimethylpropyl oder Phenyl bedeutet,
R⁶, R⁷ unabhängig voneinander jeweils eine Alkylgruppe mit 1 bis 10 Kohlenstoffatomen, bevorzugt 2 bis 6, noch bevorzugter 1 bis 4 Kohlenstoffatomen sind und
R⁸ Wasserstoff oder eine Alkylgruppe mit 1 bis 10 Kohlenstoffatomen, bevorzugt Wasserstoff oder eine Alkylgruppe mit 1 bis 6 Kohlenstoffatomen, noch bevorzugter Wasserstoff oder eine Alkylgruppe mit 1 bis 4 Kohlenstoffatomen ist.

Die Radikalbildner der Formel **(V)** sind an sich bekannt und zum Teil kommerziell erhältlich.

Der Radikalbildner der Formel **(V)** ist dabei vorzugsweise gewählt aus der Gruppe bestehend aus *tert*-Butylperoxypivalat, *tert*-Amylperoxypivalat, *tert*-Butylperoxyneodecanoat, 1,1,3,3-Tetramethylbutylperoxyneodecanoat, *tert*-Butylperoxy-2-ethylhexanoat, 1,1,3,3-Tetramethylbutylperoxy2-ethylhexanoat, *tert*-Amylperoxyneodecanoat, Cumylperoxyneodecanoat, Cumylperoxyneoheptanoat, Cumylperoxypivalat, und deren Mischungen.

Der Radikalbildner der Formel **(V)** ist dabei bevorzugt gewählt aus der Gruppe bestehend aus *tert*-Butylperoxyneodecanoat, 1,1,3,3-Tetramethylbutylperoxyneodecanoat, *tert*-Butylperoxy-2-ethylhexanoat, 1,1,3,3-Tetramethylbutylperoxy-2-ethylhexanoat, Cumylperoxyneodecanoat, und deren Mischungen, dabei wiederum besonders bevorzugt sind 1,1,3,3-Tetramethylbutylperoxyneodecanoat, *tert*-Butylperoxyneodecanoat und/oder *tert*-Butylperoxy-2-ethylhexanoat.

Insbesondere die als bevorzugt angegebenen Radikalbildner ermöglichen eine sehr gute Reaktionsführung unter milden Reaktionsbedingungen, insbesondere in dem als bevorzugt angegebenen Temperaturbereich, wodurch die gewünschten Verbindungen der Formel **(III)** in hohen Ausbeuten und hoher Reinheit erhalten werden können.

Vorzugsweise werden in Schritt c) des erfindungsgemäßen Verfahrens insgesamt 0.1 bis 10 mol-%, bevorzugt 0.25 bis 7 mol-%, weiter bevorzugt 0.5 bis 7 mol%, insbesondere bevorzugt 0.5 bis 5 mol%, an Radikalbildnern der Formel **(V)** verwendet, bezogen auf die insgesamt in Schritt c) des erfindungsgemäßen Verfahrens eingesetzte, vom Reinprodukt umfasste Menge aller Verbindungen der Formel **(I)**.

Die weiteren bevorzugten Bedingungen der Umsetzung in Schritt c) sind dem Fachmann bekannt und können etwa der WO 2015/173146 A1 entnommen werden.

Die nachfolgenden Beispiele sollen die Erfindung erläutern, ohne diese zu beschränken.

### Beispiele.

### 1. Herstellung von Roh-AcCH (Schritt a)

1.1 50 g Acrolein werden mit 1.3 Mol-Äquivalenten Blausäure und soviel Triethylamin, dass der pH der Mischung bei 7.3 liegt, als Flüssigkeit in einem Doppelmantelreaktor bei - 5 °C unter Rührung vermischt und umgesetzt. Die Dosierung der Edukte und des Katalysators erfolgt kontinuierlich über 2 Stunden, um die Exothermie zu begrenzen. Nach 2 Stunden Reaktionszeit wird die Reaktionsmischung mit einer Mischung von para-Toluolsulfonsäure und Essigsäure (Gewichtsverhältnis 1 : 1) versetzt, bis der pH unter pH 3 absinkt. Dieses Rohprodukt umfasst ∼ 30 Mol.-% Blausäure bezogen auf Acroleincyanhydrin.

1.2 Zum Vergleich wird versucht, Roh-AcCH auch über das in der US 3,850,976, Beispiel 1 beschriebene Verfahren zu erhalten. Dieses Verfahren enthält allerdings einen Schritt der Destillierung von AcCH unter reduziertem Druck (65 °C, 3mm). Dieser Schritt ist unter Laborbedingungen beherrschbar, aber für größere Ansätze nicht durchführbar. Dieses Verfahren scheidet für großtechnische Ansätze deshalb aus und stellt auch für kleine Ansätze ein Sicherheitsrisiko dar.

### 2. Reinigung des Rohproduktes aus 1 (Schritt b)

Das AcCH-Rohprodukt, welches nach dem Schritt 1.1 erhalten wird, wird mit Hilfe einer Telabpumpe und eines externen Thermostaten auf eine Temperatur von 30 - 72.5 °C vorgewärmt und in Mengen von 10 - 50 g/h auf eine 30 cm lange, beheizbare Strippkolonne auf den Kopf der Kolonne gefahren werden. Die Kolonne wird unter Anlegen eines Vakuums mit einem Druck von 10 - 200 mbar gefahren.

Die Kolonne enthält gemäß dem Stand der Technik ein Metallgewebe, was die in der Produktion verwendeten geregelten Packungen ausreichend simuliert.

Erfahrungsgemäß entsprechen 10 cm dieses Metallgewebes 5 theoretischen Böden.
Die Kolonne wird beheizt, aber nur um Wärmeverluste zu kompensieren und die Kolonne bei der genannten Temperatur zu halten.

Auf der Kolonne befindet sich ein Rücklaufteiler, mit dem Verhältnis von Rücklauf und Abnahme eingestellt werden kann. Integriert ist außerdem ein Kondensator, mit dem Brüden kondensiert werden. Die Brüden bestehen in diesem Falle im Wesentlichen aus kondensierter Blausäure. Der Destillatvorlagebehälter kann Phosphorsäure enthalten, um eine ausreichende Stabilisierung der kondensierten Blausäure sicherzustellen.

Im Laborverfahren wird die Blausäure mit wässriger Natronlauge neutralisiert und einer geregelten Entsorgung zugeführt.

Im späteren Produktionsverfahren kann diese verbrannt oder recycliert werden.

Das blausäurefreie Acroleincyanhydrin wird mit der gleichen Menge pro Zeit, wie der Feed auf die Kolonne, mit Hilfe einer Pumpe ausgeschleust. Dadurch bleibt der Sumpfstand konstant und die thermische Belastung des Materials ist zeitlich limitiert.

Die Beheizung der Destillation erfolgt über einen Doppelmantel der Sumpfblase.

Das blausäurefreie Acroleincyanhydrin im Sumpf wird per argentometrischer Titration mit Silbernitratlösung bezüglich der resliche Cyanidspuren analysiert.

Es zeigt sich eine äußerst geringe Menge von weniger als 0.1 Gew.-% an Blausäure bezogen auf Acroleincyanhydrin im so aufgereinigten Reinprodukt.

Es wird festgestellt, dass bei Ansteigen des Druckes ≥ 1 bar eine Reinigung des AcCHs im großtechnischen Maßstab nicht umsetzbar ist, da es zu unkontrollierten thermischen Zersetzungen kommt. Diese sind im Druckbereich von 10 - 200 mbar besonders gut beherrschbar.

## Patentansprüche

1. Verfahren zur Herstellung einer Verbindung der Formel **(I)** 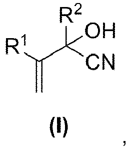 wobei R¹ und R² Wasserstoff sind,
umfassend die folgenden Schritte:
(a) Umsetzen mindestens einer Verbindung der Formel **(II)** 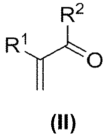 mit Blausäure und mindestens einer Base **B** ausgewählt aus der Gruppe bestehend aus Ammoniak, Trialkylamin, Ammoniumsalz, wodurch ein Rohprodukt **RP**, welches **(I)** und Blausäure umfasst, erhalten wird;
b) mindestens teilweise Entfernung von Blausäure aus dem Rohprodukt **RP**, in dem dieses einer Strippung unterworfen wird, wodurch ein Reinprodukt umfassend die Verbindung **(I)**, wobei das Reinprodukt einen gegenüber **RP** verringerten Gehalt an Blausäure aufweist, erhalten wird,
**dadurch gekennzeichnet, dass** die Strippung bei einem Druck im Bereich von 10 bis 500 mbar durchgeführt wird, wobei bei der Strippung in Schritt b) eine Temperatur von ≤ 85 °C eingehalten wird, wobei gegebenenfalls ein inertes Strippungsgas im Gegenstrom eingesetzt wird.

2. Verfahren nach Anspruch 1, wobei Schritt a) bei einer Temperatur von - 50 °C bis 80 °C durchgeführt wird.

3. Verfahren nach Anspruch 1 oder 2, wobei die Blausäure in Schritt a) in einer Menge von > 1 molaren Äquivalent, bezogen auf die Menge aller in Schritt a) eingesetzten Verbindungen der Formel **(II)**, eingesetzt wird.

4. Verfahren nach einem der Ansprüche 1 bis 3, wobei die Menge an Base **B** in Schritt a) 0.01 bis 5 Gew.-%, bezogen auf die Summe der Gewichte der in Schritt a) eingesetzten Blausäure und aller in Schritt a) eingesetzten Verbindungen der Formel **(II)**, beträgt.

5. Verfahren nach einem der Ansprüche 1 bis 4, wobei das Rohprodukt **RP** vor dem Schritt b) mit mindestens einer Säure vermischt wird, so dass es nach Vermischen mit der Säure einen pH-Wert von < 6.9 aufweist.

6. Verfahren nach Anspruch 5, wobei mindestens eine Säure einen pKs von ≤ 1 hat.

7. Verfahren nach Anspruch 5 oder 6, wobei die Säure ausgewählt ist aus Mineralsäure, Alkylcarbonsäure, aromatische Sulfonsäure.

8. Verfahren nach einem der Ansprüche 1 bis 7, wobei das Rohprodukt **RP** in Schritt b) bei der Strippung mindestens teilweise über eine Schüttung aus Füllkörpern oder über eine strukturierte Packung geleitet wird.

9. Verfahren zur Herstellung einer Verbindung der Formel **(III)** 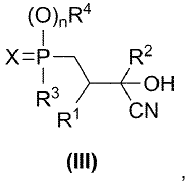
wobei R¹ und R² die vorgenannten Bedeutungen haben,
R³, R⁴ unabhängig voneinander jeweils ausgewählt aus (Halo)alkyl, (Halo)aryl, (Halo)aralkyl, (Halo)cycloalkyl sind,
X Sauerstoff oder Schwefel ist,
n = 0 oder 1 ist;
umfassend die Schritte a) und b) gemäß einem der Ansprüche 1 bis 8 und zusätzlich den Schritt c), in welchem das in Schritt b) erhaltene Reinprodukt umfassend die Verbindung **(I)**, wobei das Reinprodukt einen gegenüber **RP** verringerten Gehalt an Blausäure aufweist, mit einer Verbindung der Formel **(IV)** 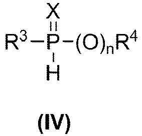 umgesetzt wird.

10. Verfahren nach Anspruch 9, wobei die Umsetzung in Schritt c) bei einer Temperatur von 50 °C bis 105 °C erfolgt.

11. Verfahren nach Anspruch 9 oder 10, wobei R³, R⁴ unabhängig voneinander jeweils ausgewählt sind aus (Halo)alkyl mit 1 bis 12 Kohlenstoffatomen, (Halo)aryl mit 6 bis 10 Kohlenstoffatomen, (Halo)aralkyl mit 7 bis 10 Kohlenstoffatomen, (Halo)cycloalkyl mit 4 bis 10 Kohlenstoffatomen.

12. Verfahren nach einem der Ansprüche 9 bis 11, wobei in Schritt c) mindestens eine radikalbildende Substanz eingesetzt wird,

13. Verfahren nach Anspruch 12, wobei die radikalbildende Substanz ein Radikalbildner der Formel **(V)** ist mit 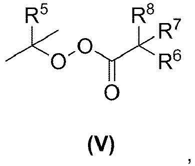
wobei R⁵ ausgewählt ist aus Methyl, Ethyl, 2,2-Dimethylpropyl, Phenyl,
R⁶, R⁷ unabhängig voneinander jeweils eine Alkylgruppe mit 1 bis 10 Kohlenstoffatomen sind,
R⁸ Wasserstoff oder eine Alkylgruppe mit 1 bis 10 Kohlenstoffatomen ist.

## Claims

1. Method for preparing a compound of the formula **(I)** 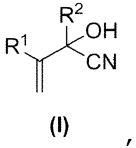 where R¹ and R² are hydrogen,
comprising the following steps:
(a) reacting at least one compound of the formula **(II)** 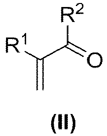 with hydrocyanic acid and at least one base **B** selected from the group consisting of ammonia, trialkylamine, ammonium salt, whereby a crude product **CP** comprising **(I)** and hydrocyanic acid is obtained;
b) at least partially removing hydrocyanic acid from the crude product **CP** by subjecting the latter to stripping, whereby a pure product comprising the compound **(I)** is obtained, wherein the pure product has a reduced content of hydrocyanic acid compared to **CP**,
**characterized in that** the stripping is carried out at a pressure in the range of 10 to 500 mbar, wherein a temperature of ≤ 85°C is maintained during the stripping in step b), wherein optionally an inert stripping gas is used in countercurrent flow.

2. Method according to Claim 1, wherein step a) is conducted at a temperature of -50°C to 80°C.

3. Method according to Claim 1 or 2, wherein the hydrocyanic acid in step a) is used in an amount of > 1 molar equivalent, based on the amount of all compounds of the formula **(II)** used in step a).

4. Method according to any of Claims 1 to 3, wherein the amount of base **B** in step a) is 0.01 to 5% by weight, based on the sum of the weights of hydrocyanic acid used in step a) and all compounds of the formula **(II)** used in step a).

5. Method according to any of Claims 1 to 4, wherein the crude product **CP** is mixed prior to step b) with at least one acid so that after mixing with the acid it has a pH of < 6.9.

6. Method according to Claim 5, wherein at least one acid has a pKa of ≤ 1.

7. Method according to Claim 5 or 6, wherein the acid is selected from mineral acid, alkylcarboxylic acid, aromatic sulfonic acid.

8. Method according to any of Claims 1 to 7, wherein the crude product **CP** during the stripping in step b) is passed at least partially over a bed of packing elements or over a structured packing.

9. Method for preparing a compound of the formula **(III)** 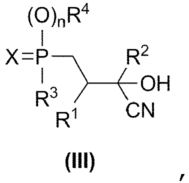
where R¹ and R² are as defined above,
R³, R⁴ are each independently selected from (halo)alkyl, (halo)aryl, (halo)aralkyl, (halo)cycloalkyl,
X is oxygen or sulfur,
n = 0 or 1;
comprising the steps a) and b) according to any of Claims 1 to 8 and additionally comprising the step c), in which the pure product comprising the compound **(I)** obtained in step b), wherein the pure product has a reduced content of hydrocyanic acid compared to **CP**, is reacted with a compound of the formula **(IV)**

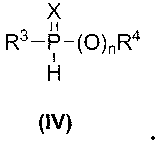

10. Method according to Claim 9, wherein the reaction in step c) is conducted at a temperature of 50°C to 105°C.

11. Method according to Claim 9 or 10, where R³, R⁴ are each independently selected from (halo)alkyl having 1 to 12 carbon atoms, (halo)aryl having 6 to 10 carbon atoms, (halo)aralkyl having 7 to 10 carbon atoms, (halo)cycloalkyl having 4 to 10 carbon atoms.

12. Method according to any of Claims 9 to 11, wherein at least one free-radical-forming substance is used in step c).

13. Method according to Claim 12, wherein the free-radical-forming substance is a free-radical former of the formula **(V)** 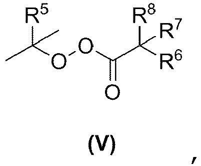
where R⁵ is selected from methyl, ethyl, 2,2-dimethylpropyl, phenyl,
R⁶, R⁷ are each independently an alkyl group having 1 to 10 carbon atoms,
R⁸ is hydrogen or an alkyl group having 1 to 10 carbon atoms.

## Revendications

1. Procédé de fabrication d'un composé de la formule **(I)** : 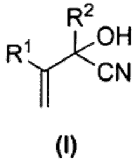 dans laquelle R¹ et R² représentent l'hydrogène,
comprenant les étapes suivantes :
(a) la mise en réaction d'au moins un composé de la formule **(II)** : 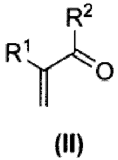 avec de l'acide cyanhydrique et au moins une base **B** choisie dans le groupe constitué par l'ammoniac, la trialkylamine, un sel d'ammonium ; un produit brut **RP**, qui comprend **(I)** et de l'acide cyanhydrique, étant obtenu ;
b) l'élimination au moins partielle de l'acide cyanhydrique du produit brut **RP**, par soumission de celui-ci à une extraction, un produit pur comprenant le composé **(I)**, le produit pur présentant une teneur réduite en acide cyanhydrique par rapport à **RP**, étant obtenu,
**caractérisé en ce que** l'extraction est réalisée à une pression dans la plage allant de 10 à 500 mbar, une température ≤ 85 °C étant maintenue lors de l'extraction à l'étape b), un gaz d'extraction inerte étant éventuellement utilisé à contre-courant.

2. Procédé selon la revendication 1, dans lequel l'étape a) est réalisée à une température de -50 °C à 80 °C.

3. Procédé selon la revendication 1 ou 2, dans lequel l'acide cyanhydrique est utilisé à l'étape a) en une quantité > 1 équivalent molaire, par rapport à la quantité de tous les composés de la formule **(II)** utilisés à l'étape a).

4. Procédé selon l'une quelconque des revendications 1 à 3, dans lequel la quantité de base **B** à l'étape a) est de 0,01 à 5 % en poids, par rapport à la somme des poids de l'acide cyanhydrique utilisé à l'étape a) et de tous les composés de la formule **(II)** utilisés à l'étape a).

5. Procédé selon l'une quelconque des revendications 1 à 4, dans lequel le produit brut **RP** est mélangé avec au moins un acide avant l'étape b), de telle sorte qu'il présente après le mélange avec l'acide un pH < 6,9.

6. Procédé selon la revendication 5, dans lequel au moins un acide a un pKs ≤ 1.

7. Procédé selon la revendication 5 ou 6, dans lequel l'acide est choisi parmi un acide minéral, un acide alkylcarboxylique, un acide sulfonique aromatique.

8. Procédé selon l'une quelconque des revendications 1 à 7, dans lequel le produit brut **RP** est acheminé à l'étape b) lors de l'extraction au moins en partie sur un lit de corps de remplissage ou sur un garnissage structuré.

9. Procédé de fabrication d'un composé de la formule **(III)** : 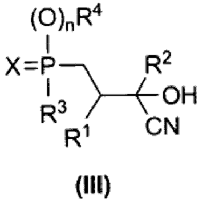
dans laquelle R¹ et R² ont les significations indiquées précédemment,
R³, R⁴ sont chacun choisis indépendamment l'un de l'autre parmi (halo)alkyle, (halo)aryle, (halo)aralkyle, (halo)cycloalkyle,
X représente l'oxygène ou le soufre,
n = 0 ou 1 ;
comprenant les étapes a) et b) selon l'une quelconque des revendications 1 à 8, et en outre l'étape c), lors de laquelle le produit pur comprenant le composé **(I)** obtenu à l'étape b), le produit pur présentant une teneur réduite en acide cyanhydrique par rapport à **RP**, est mis en réaction avec un composé de la formule **(IV)** :

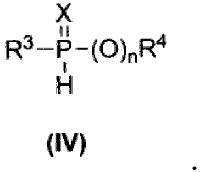

10. Procédé selon la revendication 9, dans lequel la réaction à l'étape c) a lieu à une température de 50 °C à 105 °C.

11. Procédé selon la revendication 9 ou 10, dans lequel R³, R⁴ sont choisis chacun indépendamment l'un de l'autre parmi (halo)alkyle de 1 à 12 atomes de carbone, (halo)aryle de 6 à 10 atomes de carbone, (halo)aralkyle de 7 à 10 atomes de carbone, (halo) cycloalkyle de 4 à 10 atomes de carbone.

12. Procédé selon l'une quelconque des revendications 9 à 11, dans lequel au moins une substance formant des radicaux est utilisée à l'étape c) .

13. Procédé selon la revendication 12, dans lequel la substance formant des radicaux est un agent de formation de radicaux de la formule **(V)** : 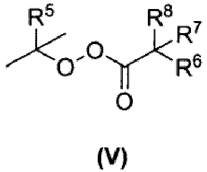
dans laquelle R⁵ est choisi parmi méthyle, éthyle, 2,2-diméthylpropyle, phényle,
R⁶, R⁷ représentent chacun indépendamment l'un de l'autre un groupe alkyle de 1 à 10 atomes de carbone,
R⁸ représente l'hydrogène ou un groupe alkyle de 1 à 10 atomes de carbone.
